# EUROPEAN PATENT SPECIFICATION

(11) **EP 4 162 063 B1**
(45) Date of publication and mention of the grant of the patent: **27.08.2025**
(21) Application number: 20775152.0
(22) Date of filing: 16.06.2020
(51) Int. Cl.: C12Q 1/02, C12Q 1/24, C12N 7/02, G01N 33/569, A61B 10/00

(54) **MICROORGANISM CONCENTRATION METHOD WITH ELASTIC POLYMERS**
VERFAHREN ZUR KONZENTRIERUNG VON MIKROORGANISMEN MIT ELASTISCHEN POLYMEREN
PROCÉDÉ DE CONCENTRATION DE MICRO-ORGANISMES AVEC DES POLYMÈRES ÉLASTIQUES

(30) Priority: 09.06.2020 TR 202008937
(43) Date of publication of application: 12.04.2023
(73) Proprietor: BIO T BIYOTEKNOLOJI ÇÖZÜMLERI VE ÜRETIM ANONIM SIRKETI, Pendik, Istanbul (TR)
(72) Inventor: KOCAGÖZ, Zühtü Tanil, Atasehir/Istanbul (TR); CAN, Özge, Atasehir/Istanbul (TR)
(74) Representative: Yamankaradeniz, Kemal
(86) International application number: PCT/TR2020/000003
(87) International publication number: WO 2021/251914

(56) References cited:
- WO-A1-2009/018544
- WO-A1-2009/018544
- WO-A1-99/05317
- WO-A1-99/05317
- KR-A- 20140 066 003
- KR-A- 20140 066 003
- US-A1- 2005 064 395
- US-A1- 2005 064 395
- US-A1- 2006 019 274
- US-A1- 2006 019 274
- WAN-TZU CHEN ET AL: "Mechanistic study of membrane concentration and recovery of Listeria monocytogenes", BIOTECHNOLOGY AND BIOENGINEERING, JOHN WILEY, HOBOKEN, USA, vol. 89, no. 3, 21 December 2004 (2004-12-21), pages 263 - 273, XP071155274, ISSN: 0006-3592, DOI: 10.1002/BIT.20256
- FERNANDEZ-GUTIERREZ MAR ET AL: "Low-density polypropylene meshes coated with resorbable and biocompatible hydrophilic polymers as controlled release agents of antibiotics", ACTA BIOMATERIALIA, vol. 9, no. 4, 1 April 2013 (2013-04-01), AMSTERDAM, NL, pages 6006 - 6018, XP093090409, ISSN: 1742-7061, DOI: 10.1016/j.actbio.2012.12.012
- GORJI MOHSEN ET AL: "Development of Highly pH-Sensitive Hybrid Membranes by Simultaneous Electrospinning of Amphiphilic Nanofibers Reinforced with Graphene Oxide", JOURNAL OF FUNCTIONAL BIOMATERIALS, vol. 10, no. 2, 21 May 2019 (2019-05-21), pages 23, XP093090419, DOI: 10.3390/jfb10020023
- HO TZU-CHUAN ET AL: "Hydrogels: Properties and Applications in Biomedicine", MOLECULES, vol. 27, no. 9, 2 May 2022 (2022-05-02), pages 2902, XP093090422, DOI: 10.3390/molecules27092902
- DSOUZA ANDREA ET AL: "Multifunctional Composite Hydrogels for Bacterial Capture, Growth/Elimination, and Sensing Applications", APPLIED MATERIALS & INTERFACES, vol. 14, no. 42, 12 October 2022 (2022-10-12), US, pages 47323 - 47344, XP093090413, ISSN: 1944-8244, DOI: 10.1021/acsami.2c08582

## Description

### Field of the invention:

The present invention is related to use of "elastic hydrophilic polymer meshes" in detection of infectious agents in a fluid biological sample by a diagnostic test for concentrating infectious agents including viruses and large molecules of all microorganisms by removing water and other small molecules with molecular weight less than 500 Daltons from the sample, thereby increasing the sensitivity of the detection of the infectious agents. The present invention can be evaluated in the fields of biology and chemistry.

### The basis of the invention:

Biological fluids from humans are often studied by various methods, for the diagnosis of infectious diseases. These methods include microscopy, culture, rapid antigen tests and nucleic acid amplification methods such as polymerase chain reaction (PCR). Regardless of the detection method, if the amount of the infectious agent in the sample is below the sensitivity limits of these tests, they cannot detect the agent. These tests, by nature, can evaluate a sample with a very small volume. However, many biological samples such as blood, urine and mouthwash can be easily taken tens of times more than the amount used in these tests. In addition, biological samples may contain substances that inhibit reactions that allow the detection of microorganisms, such as PCR, by amplifying their nucleic acids. The aim of this present invention is to concentrate infectious agents with elastic polymer molecular meshes, which reduce the volume of biological samples by removing water and other small molecules from the environment, as well as to eliminate small molecules that inhibit the reactions of diagnostic methods, thereby increasing the sensitivity of these tests.

### Detailed description of the invention and the difference of the technique from known methods:

Concentration of infectious agents in liquid biological samples increases the sensitivity of diagnostic methods.

The most commonly used method, to increase the detection sensitivity of infectious agents in liquid biological samples by diagnostic tests, is the concentration of microorganisms by centrifuging the liquids. After microorganisms are sedimented by centrifuging, the supernatant is poured and the organisms are concentrated in a small amount of liquid remaining at the bottom of the tube. The chance of detecting organisms such as bacteria and fungi, sought in the microscopic examination of this concentrated liquid, is higher compared to the original sample. It is also more likely to detect the organism by other diagnostic methods such as culture, molecular diagnostic methods and antigen tests, from a concentrated sample.

Centrifugation is a time-consuming process. It also requires a centrifuge with appropriate features. In laboratories without centrifuge, this concentration process cannot be applied. In order to concentrate the samples, it should be placed in the appropriate centrifuge tube, placed in the centrifuge device and generally rotated for several minutes. Then the supernatant is removed. There is a risk of transmission of organisms that cause disease, during these processes. Although the bacteria, fungi and parasites can be concentrated by centrifuging, the viruses cannot be concentrated directly by this way, as they are much smaller than these organisms.

Another method of concentrating infectious agents in biological fluids is by filtration. This process is used to concentrate microorganisms such as bacteria and fungi that cause contamination in foods such as milk. Filtration process requires special filters with 0.2-0.4 micron pore diameter. Since bacteria and fungi are larger than these pores, they cannot pass through the filter and are concentrated on the filter. Viruses, on the other hand, are very small and easily pass through such filter pores and cannot be condensed by filtration method. For this reason, both centrifugation and filtration are not suitable methods for condensing disease-causing viruses from biological liquid samples.

The present invention that we describe here is use of "elastic hydrophilic polymer meshes" in detection of infectious agents in a fluid biological sample by a diagnostic test for concentrating infectious agents, including viruses, large molecules of all microorganisms by removing water and other small molecules with molecular weight less than 500 Daltons from the sample, thereby increasing the sensitivity of the detection of the infectious agents. Three-dimensional cage-like structures made up of elastic polymers are used to incorporate small molecules, while the volumes of the mesh structures expand. Meanwhile, the size of the pores on their surface allow organisms to concentrate in a small amount of fluid outside, by not allowing any infectious agents, including viruses, and their large antigens to enter this three-dimensional structure. The fluid containing the infectious agents outside these structures is taken from there and then used in diagnostic tests such as microscopy, culture, nucleic acid amplification and immunological tests. For example, the sensitivity of nucleic acid amplification methods such as PCR, used to detect viral and bacterial agents such as SARS-CoV2 (new coronavirus), Influenza (flu virus), *Mycobacterium tuberculosis,* (tuberculosis bacillus), *Streptococcus pyogenes* can be increased as a result of concentration of infectious agents in mouthwash. Similarly, the sensitivity of antigen tests, microscopy and culture methods can also be increased. The sensitivity of the tests used in the diagnosis of microorganisms causing infections in central nervous system infection, can be increased by concentrating the cerebrospinal fluid and infectious agents causing urinary tract infections, by concentrating urine, using this new method. This new concentration method also contributes to the increased sensitivity of diagnostic tests, as molecules of small structures, which are present in biological fluids and inhibit the reactions of PCR and antigen tests, are also removed during the concentration process.

Regis Peytavi et al. in their patent application numbered US 8481265 B2 and named "Concentration and Enrichment of Microbial Cells and Microbial Nucleic Acids from Bodily Fluids" and in their equivalent applications made in other countries, stated that centrifugation or filtration methods are used for the concentration of microorganisms (Claim 14, item b). In this application the concentration method described is for bacteria and it is not possible to concentrate viruses by this method.

Apart from the classical filtration and centrifugation methods as described above, other microorganism concentration methods have been defined. These methods, which differ from the invention we have described above, and their different aspects, are described below.

In the patent application made by Kshirsagar, Manjiri T. et al. EP 2 205 717 B1 named "Microorganism Concentration Process", and in their equivalent applications made in many countries, they describe a method of concentrating the microorganisms, by binding microorganisms to metal silicate crystals such as gamma-FO(OH) (iron oxide) and separating them from the liquid. In this method, it is not possible to reduce the liquid volume by removing water and other small molecules, thus concentrate microorganisms in a small volume, as we have described above in our new method. In patent application WO 2013/184186 named "Bismuth-containing Concentrating Agents for Microorganism" and similar applications made in various countries of the same application made by the same researchers, in order to concentrate microorganisms, it has been proposed using bismuth salts instead of gamma-FO(OH). Also, in their other application named "Microorganism concentration agent and method of making" - US 2014/001 1253 A1 they proposed using titanium dioxide, gold or platinum, bonded to silica sand for the binding of microorganisms, or in patent US 9,575,059 B2 named "Lanthanum-based Concentration Agents" they proposed using lanthanum carbonate. In these applications, it is not possible to reduce the liquid volume by removing the small molecules by polymers as we defined in our invention in this document.

A similar patent application has been filed by Philip T. Feldsine to concentrate microorganisms. In patent application numbered US 2005/0123954 A1 and named as "Methods Compositions, and Kits for the concentration and detection of Microorganisms", it is proposed that microorganisms are attached to various surfaces and concentrated by separating them from liquids. This is also not similar to the use of elastic hydrophilic polymer meshes that we described in this document.

In the patent application WO2009018544A1 named "Pathogen detection in large-volume particulate samples" discloses filter systems for detecting microorganisms in a large-volume particulate samples such as a food sample. However, the principle of this system is filtration not concentration by removing water and small molecules from the fluid samples we described in this document. These filtration systems cannot concentrate viruses, proteins and nucleic acids as the invention we described in this document.

In a study of WAN-TZU CHEN ET AL named "Mechanistic study of membrane concentration and recovery of Listeria monocytogenes" describes concentration method by filtering liquids through a membrane with small pores. The polymers mentioned here, such as cellulose and PVDF, are the polymers used to create membranes. It has a filter feature that retains the molecules and microorganisms in the liquids passed from one side to the other. The polymer network in this invention has a two-dimensional structure like a paper. However this document does not discloses a use in accordance with claim described in this document.

The patent application WO9905317A1 named "Method for purifying viral nucleic acids" relates to a method for purification of viral RNA from a biological sample. The method involves lysing the virus envelope to liberate the RNA and passing the lysate through a porous hydrophilic PVDF filter to capture the viral RNA. This is also not similar to the use of elastic hydrophilic polymer meshes that we described in this document.

The patent application US2005064395A1 named "Liquid crystal based analyte detection" relates to the field of detection of viruses, and in particular to detection of viruses using a liquid crystal assay format. However this document does not disclose a use in accordance with claim described in this document.

In the patent application KR20140066003A named "Entrapment matrix enhancing internal mass transfer, and biosensor using the same, and preparing method of the same" describes an immobilization insoluble carrier having improved substance transferring ability to the inside, and a biosensor using the same and a preparation method thereof. However this document does not disclose a use in accordance with claim described in this document.

In the patent application US2006019274A1 named "Nano-PCR: methods and devices for nucleic acid amplification and detection" relates methods, devices, and compositions that provide for amplification of nucleic acid sequences without reliance upon temperature cycling, thus freeing the methods from conventional benchtop thermal cycling devices. This is also not similar to the use of elastic hydrophilic polymer meshes that we described in this document.

None of the methods, we cited above use of "elastic hydrophilic polymer meshes" in detection of infectious agents in a fluid biological sample by a diagnostic test, for concentrating infectious agents including viruses and large molecules of all microorganisms that we describe in this application. In patent document CNA031294081A, named "Protein, Fast Method Virus Concentration" a method based on co-precipitation of viruses bound to albumin is described. Similarly, in patent application US 3470067 named "Concentration and Purification of Viruses from Particulate Magnetic Iron Oxide-Virus Complexes", viruses are attached to magnetic iron oxide particles and concentrated. In the patent application CN 100509656C and named "Method for Concentrating Virus in Sewage or Sewage Treatment Plant Tail Water", the concentration method defined is proposing binding of viruses to silica gel in the presence of Al³⁺ and separation after washing with sulfuric acid. All of these methods are based on concentration by binding viruses to a surface or by bonding and sedimentation together with various molecules, and they are different methods from volume reduction by removing small molecules from the environment with the elastic polymer meshes as we describe in this application. In the method we describe, microorganisms are not bound to any surface or molecule. Reducing the volume of liquid and concentrating viruses and other microorganisms is achieved by removing the water and other small molecules surrounding the viruses that are freely present in the liquid, with elastic polymers.

In patent application CN103923883A named "Concentration and Purification Method for Influenza Virus" the concentration method of viruses defined is size exclusion chromatography, which is a different method than the method we describe in this application.

### Examples:

1- Suspensions of bacteria, *Escherichia coli, Staphylococcus aureus, Candida albicans* and *Mycobacterium tuberculosis* H37Ra, were prepared in sterile 0.9% NaCl solution from fresh cultures. 10 ml of the suspensions were placed in a sterile tube, and previously synthesized and dried elastic polymers were added into the suspensions and waited for 5 minutes for concentration. 1/10 serial dilutions were made by using 100 microliters of unconcentrated and concentrated liquids, again using 0.9% NaCl. Then, 10 microliters of *Escherichia coli* and *Staphylococcus aureus* dilutions were inoculated to Mueller Hinton Agar, dilutions of *Candida albicans* to Sabouraud Dextrose Agar and *Mycobacterium tuberculosis* H37Ra dilutions to Löwenstein-Jensen media. Mueller Hinton and Sabouraud Dextrose Agar media were kept for 2 days and Löwenstein-Jensen media for 3 weeks in a 37°C incub ator. At the end of these periods, the number of colony forming units per milliliter of bacterial suspension was determined by counting the colonies formed in the media. An increase of 20 to 40-fold in samples concentrated by elastic polymers, relative to the dilute suspensions of all microorganisms tested was determined.
2- Samples that will be tested were prepared by adding SARS-CoV2 (new coronavirus) which were grown in Vero cells and then inactivated, into viral transport medium. In these samples, SARS-CoV2 RNA was investigated with Real-Time PCR before and after concentration by elastic polymers after extraction of RNA. It was identified that in samples concentrated with the polymer, SARS-CoV2 RNA was detected 4 to 6 cycles earlier, which indicated that the amount of viruses in these samples was increased by 16 to 64 times.

We can conclude that this use of "elastic hydrophilic polymer meshes" in detection of infectious agents in a fluid biological sample by a diagnostic test, for concentrating infectious agents including viruses and large molecules of all microorganisms, has several advantages over previously described methods such as being a rapid, easy and effective method.

## Claims

1. Use of elastic hydrophilic polymer meshes in detection of infectious agents in a liquid biological sample by a diagnostic test, for concentrating infectious agents including viruses and large antigens of all microorganisms by removing water and other small molecules with molecular weight less than 500 Daltons from the sample, thereby increasing the sensitivity of the detection of the infectious agents.

2. The use of claim 1, **characterized in that**; said "elastic hydrophilic polymer meshes" are made of all kinds of hydrophilic polymers, preferably polyacrylamide or polyacrylic acid or polymethacrylate and its derivatives or dextran or agar or agarose or gelatin or glycogen.

3. The use of claim 1 or 2, wherein the diagnostic test is preferably antigen and antibody detection by immunological tests including immunochromatographic tests or Enzyme Linked Immunosorbent Assay (ELISA); or nucleic acid amplification tests including polymerase chain reaction (PCR), loop-mediated amplification (LAMP), strand displacement amplification (SDA) and rolling circle amplification; or microscopic examination or culture.

## Patentansprüche

1. Verwendung von elastischen hydrophilen Polymernetzen bei Detektion von infektiösen Erregern in einer flüssigen biologischen Probe durch einen diagnostischen Test, um infektiöse Erreger, einschließlich Viren und großer Antigene aller Mikroorganismen, durch Entfernen von Wasser und anderen kleinen Molekülen mit einem Molekulargewicht von weniger als 500 Dalton aus der Probe zu konzentrieren, wodurch die Empfindlichkeit der Detektion der infektiösen Erreger erhöht wird.

2. Verwendung nach Anspruch 1, **dadurch gekennzeichnet, dass**; die "elastischen hydrophilen Polymernetze" aus allen Arten von hydrophilen Polymeren, vorzugsweise Polyacrylamid oder Polyacrylsäure oder Polymethacrylat und seinen Derivaten oder Dextran oder Agar oder Agarose oder Gelatine oder Glykogen gefertigt sind.

3. Verwendung nach Anspruch 1 oder 2, wobei der diagnostische Test vorzugsweise eine Antigen- und Antikörperdetektion durch immunologische Tests, einschließlich immunchromatographischer Tests oder Enzymimmunoassay (ELISA); oder Nukleinsäure-Amplifikationstests, einschließlich Polymerase-Kettenreaktion (PCR), Schleifen-vermittelte Amplifikation (LAMP), Strangverdrängungsamplifikation (SDA) und Rolling-Circle-Amplifikation; oder mikroskopische Untersuchung oder Kultur ist.

## Revendications

1. Utilisation de mailles polymères hydrophiles élastiques dans la détection d'agents infectieux dans un échantillon biologique liquide par un test de diagnostic, pour concentrer des agents infectieux, y compris des virus et des grands antigènes de tous les micro-organismes en éliminant l'eau et d'autres petites molécules de poids moléculaire inférieur à 500 Daltons de l'échantillon, augmentant ainsi la sensibilité de la détection des agents infectieux.

2. Utilisation de la revendication 1, **caractérisée en ce que** ; lesdites « mailles polymères hydrophiles élastiques » sont constituées de toutes sortes de polymères hydrophiles, de préférence de polyacrylamide ou d'acide polyacrylique ou de polyméthacrylate et de ses dérivés ou de dextrane ou d'agar-agar ou d'agarose ou de gélatine ou de glycogène.

3. Utilisation de l'une des revendications 1 ou 2, dans laquelle le test de diagnostic est de préférence une détection d'antigène et d'anticorps par des tests immunologiques comprenant des tests immunochromatographiques ou un dosage immuno-enzymatique (ELISA) ; ou des tests d'amplification d'acide nucléique, comprenant la réaction en chaîne par polymérase (PCR), l'amplification médiée par boucles (LAMP), l'amplification par déplacement de brin (SDA) et l'amplification en cercle roulant ; ou un examen microscopique ou une culture.
